# EUROPÄISCHE PATENTSCHRIFT

(11) **EP 3 804 813 B1**
(45) Veröffentlichungstag und Bekanntmachung des Hinweises auf die Patenterteilung: **08.05.2024**
(21) Anmeldenummer: 19202526.0
(22) Anmeldetag: 10.10.2019
(51) Int. Cl.: A61Q 1/02, A61K 8/04, A61K 8/81, A61K 8/85, A61K 8/87

(54) **ABDECKZUSAMMENSETZUNG**
COVERING COMPOSITION
COMPOSITION DE REVÊTEMENT

(43) Veröffentlichungstag der Anmeldung: 14.04.2021
(73) Patentinhaber: Frike Aerosol AG, 8752 Näfels (CH)
(72) Erfinder: Czech, Nikolai, 8102 Oberengstringen (CH); Jufer, Michael, 8880 Walenstadt (CH); Schwagerus, Andreas, 8867 Niederurnen (CH)
(74) Vertreter: Schneiders & Behrendt Bochum

(56) Entgegenhaltungen:
- WO-A1-2013/045379
- WO-A1-2018/122014
- DE-U1-202018 100 940
- US-A1- 2003 053 976
- US-A1- 2004 191 435
- US-A1- 2018 000 721

## Beschreibung

Die Erfindung betrifft eine Abdeckzusammensetzung zur Aufbringung auf Körperoberflächen, in Form eines Abdecksprays.

Abdeckmassen zur Aufbringung auf die Haut etwa zur Tönung oder Überdeckung von Hautdefekten in Form von Salben sind seit Jahrtausenden bekannt. Sie enthalten häufig Pigmente, die dazu dienen, der Haut eine gewünschte Färbung zu verleihen, sowie eine Trägermatrix (Salbengrundlage) und gegebenenfalls Duftstoffe.

Abgesehen von Körperpflegeprodukten für den persönlichen Bedarf gibt es einen speziellen Bedarf im Bereich der darstellenden Künste, in dem spezielle optische Effekte verlangt werden, beispielsweise eine spezielle Farbgebung oder Glimmereffekte. Häufig ist es auch erwünscht oder nötig, Tattoos, Akne, Narben oder Pigmentschäden zu kaschieren.

Herkömmliche Abdeckmassen etwa auch Theaterschminke, haben den Nachteil, dass sie nicht wasser- und abriebfest sind. Schweiß, aber auch Regen und gegebenenfalls hohe Luftfeuchtigkeit, führen dazu, dass sich die Abdeckmasse von der Haut löst und verschmiert. Bei Körperkontakt oder Kontakt mit Gegenständen löst sich der Auftrag und überträgt sich auf die Kontaktfläche. Nicht nur im Bereich der darstellenden Künste ist ein vorzeitiger Verlust der Schminke höchst unerwünscht.

Die US 2018/000721 A1 beschreibt eine wasserabweisende topische Zusammensetzung mit einem Filmbildner und einem hydrophoben festen Zusatz in Form von Partikeln. Aus der US 2003/0053976 A1 geht eine kosmetische Zusammensetzung aus in einem Latex dispergierten Pigmenten hervor.

Aufgabe der Erfindung ist es, eine Abdeckzusammensetzung bereitzustellen, die im Wesentlichen wasserfest und abriebfest ist. Gleichzeitig soll diese Abdeckzusammensetzung auf der Körperoberfläche über längere Zeit haften, ohne Alterungserscheinungen, etwa Risse, zu zeigen. Die Abdeckzusammensetzung soll zudem gut decken und farblich auf den gewünschten Ton einstellbar sein. Schließlich soll sie auch rückstandslos entfernbar sein.

Diese Aufgabe wird mit einer Abdeckzusammensetzung der eingangs genannten Art gelöst, die mindestens ein wasserfestes Polymer in einer Menge von 1 bis 20 Gewichtsprozent, mindestens ein Pigment in einer Menge von 2 bis 25 Gewichtsprozent, mindestens ein Lösungsmittel für das wasserfeste Polymer in einer Menge von 40 bis 80 Gewichtsprozent, gegebenenfalls mindestens ein Treibmittel in einer Menge von bis zu 40 Gewichtsprozent und bis zu 40 Gewichtsprozent Zusatzstoffe enthält. Die Prozentangaben sind jeweils auf die gesamte Zusammensetzung bezogen.

Die Abdeckzusammensetzung enthält als wasserfestes Polymer Polyisobutylen. Das Lösungsmittel ist ausgewählt aus Aceton, Isopropanol, Pentan, Hexan, Heptan und/oder Methylal.

Es hat sich gezeigt, dass die erfindungsgemäße Abdeckzusammensetzung die Körperoberfläche gut und längerfristig überdeckt und es damit erlaubt, Tattoos, Akne, Narben oder Pigmentschäden zu überdecken. Im Bereich der darstellenden Künste können zudem Hauttönungen generiert werden. Der Vorteil ist, dass die applizierte Schicht, deckend, wasserfest und abriebfest ist. Es findet keine Übertragung von unerwünschten Spuren oder Partikeln auf Kleider, Einrichtungsgegenstände oder andere Personen statt. Außerdem führen Reibung, Schweiß oder Wasser nicht zu einer Abnahme der Deckkraft.

Das Auftragen des Produkts auf die Haut, Haare oder Lippen, insbesondere aber auf die Haut, kann auf verschiedene Weise erfolgen, wie beispielsweise mittels Tropfen, Tupfen, Gießen, Sprühen oder Streichen. Das Produkt kann als Gel, Creme, Roll-on, Lotion, Puder oder Spray vorliegen. Besonders bevorzugt sind Sprays, insbesondere solche, die aus Druckdosen ausgebracht werden.

Die applizierte Schicht trocknet auf der Haut ausgesprochen schnell und bildet eine wasserfeste, deckende und abriebfeste Oberfläche.

Das wasserfeste Polymer ist Polyisobutylen. Polyisobutylen bildet besonders stabile und rissfeste Oberflächen aus. Geeignete Polyisobutylene sind beispielsweise die von der BASF S. A. unter der Marke Oppanol vertriebenen Produkte. Die erfindungsgemäße Zusammensetzung enthält 1 bis 20 Gewichtsprozent Polymer und insbesondere 2 bis 10 Gewichtsprozent in Form von Polyisobutylen

Die Polymere können in einem weiten Molekulargewichtsbereich eingesetzt werden, etwa mit einem mittleren Molekulargewicht nach Gewichtsmittel von 5.000 bis 5.000.000 und insbesondere von 35.000 bis 100.000.

Als Pigmente können übliche in der Kosmetik verwandte weiße oder farbige Pigmente eingesetzt werden. Infrage kommen organische wie anorganische Pigmente, insbesondere aber anorganische Pigmente wie Eisenoxid, Titandioxid und Mica, die jeweils für sich oder in Mischung verwandt werden können. Die Pigmente können in Pulverform wie auch als Flakes verwandt werden und haben in der Regel eine Teilchengröße unterhalb 800 µm (Ausschlussgrenze). Mica ergibt einen Glimmereffekt. Durch Zumischung von Metallpulver, etwa Aluminiumspulver, können metallische Effekte erzielt werden. Die erfindungsgemäße Zusammensetzung enthält 2 bis 25 Gewichtsprozent Pigmente und insbesondere 3 bis 10 Gewichtsprozent.

Schimmernde, hautfarbene Oberflächen können beispielsweise mit einer an und für sich bekannten Mischung aus 41 bis 61 % Mica, 25 bis 37 % Titandioxid und 14 bis 22 % Eisenoxid, jeweils nach Gewicht, erzielt werden. Solche Mischungen haben beispielsweise eine mittlere Teilchengröße (D 50) im Bereich von 3,0 bis 10,0 µm nach der Laser-Diffraktionsmethode.

Als Lösungsmittel kommen solche infrage, die gut hautverträglich sind. Dies sind Aceton, Isopropanol, Pentan, Hexan, Heptan und Methylal. Niedrigsiedende Kohlenwasserstoffe sind bevorzugt. Für die Aufbringung von Zusammensetzungen auf Basis von Polyisobutylen haben sich Hexan und Heptan bewährt. Selbstverständlich können Mischungen eingesetzt werden. Der Gehalt an Lösungsmittel liegt im Bereich von 40 bis 80 Gewichtsprozent. Bei Sprayformulierung liegt der Lösungsmittelanteil im oberen Bereich, bei Cremes, Gelen und Lotionen im unteren Bereich.

Erfindungsgemäße Abdeckzusammensetzung werden vorzugsweise als Spray verwandt. Infrage kommen selbstverständlich Pumpsprays, die ohne die Verwendung von Treibgasen auskommen. Bevorzugt aber sind Sprays aus Druckdosen, die unter der Verwendung von Treibgasen ausgebracht werden, etwa von Ethan, Propan, Butan, Dimethylether, Luft, Stickstoff, Distickstoffoxid oder beliebiger Mischungen davon.

Eine sprühfähige Formulierung hat beispielsweise einen Gehalt von
1 bis 20 Gewichtsprozent Polyisobutylen,
2 bis 25 Gewichtsprozent Pigment,
40 bis 80 Gewichtsprozent Heptan,
10 bis 40 Gewichtsprozent Treibmittel und
bis zu 10 Gewichtsprozent Zusatzstoffe, insbesondere Duftstoffe.

Bevorzugt ist Polyisobutylen, gelöst in Heptan. Ein Duftstoffanteil von 0,02 Gewichtsprozent ist in der Regel ausreichend.

Für die Applikation von erfindungsgemäßen Zusammensetzungen auf Basis von Polyisobutylen haben sich als Treibgase insbesondere Mischungen von Propan und Butan bewährt, da sie gleichzeitig als Lösungsmittel für das Polymer dienen.

Für die Applikation von erfindungsgemäßen Zusammensetzungen in Form von Salben, Cremes oder Gelen enthalten die Zusammensetzungen übliche Salbengrundlagen und/oder Verdickungsmittel, jedoch keine Treibmittel. Im Übrigen können die erfindungsgemäßen Zusammensetzungen Duftstoffe, Wachse, Emulgatoren, Tenside, Verdickungsmittel, Aktivstoffe, Pflegemittel, Puffersubstanzen, Konservierungsmittel und hautfreundliche Extrakte sowie Mischungen derselben enthalten.

Die erfindungsgemäßen Abdeckzusammensetzungen halten in der Regel mehrere Tage, ohne dass Risse oder sonstige Fehler entstehen. Ist eine Entfernung erforderlich, kann das jeweils eingesetzte Lösungsmittel dazu verwandt werden.

## Patentansprüche

1. Abdeckzusammensetzung in Form eines Sprays zur Aufbringung auf Körperoberflächen, enthaltend
- mindestens ein wasserfestes Polymer in einer Menge von 1 bis 20 Gew.-%,
- mindestens ein Pigment in einer Menge von 2 bis 25 Gew.-%,
- mindestens ein Lösungsmittel für das wasserfeste Polymer in einer Menge von 40 bis 80 Gew.-%,
- bis zu 40 Gew-% Treibmittel,
- bis zu 40 Gew.-% Zusatzstoffe,
wobei die Gewichtsangaben auf die gesamte Zusammensetzung bezogen sind, **dadurch gekennzeichnet, dass** sie als wasserfestes Polymer Polyisobutylen enthält und dass das Lösungsmittel ausgewählt ist aus Aceton, Isopropanol, Pentan, Hexan, Heptan und/oder Methylal.

2. Abdeckzusammensetzung nach einem der vorstehenden Ansprüche, **dadurch gekennzeichnet, dass** das Polyisobutylen ein mittleres Molekulargewicht nach Gewichtsmittel von 5.000 bis 5.000.000 aufweist.

3. Abdeckzusammensetzung nach Anspruch 2, **dadurch gekennzeichnet, dass** das Polyisobutylen ein mittleres Molekulargewicht von 35.000 bis 100.000 aufweist.

4. Abdeckzusammensetzung nach einem der vorstehenden Ansprüche, **dadurch gekennzeichnet, dass** es Pigmente auf Basis von Eisenoxid, Titandioxid und/oder Mica enthält.

5. Abdeckzusammensetzung nach Anspruch 6, **dadurch gekennzeichnet, dass** die Pigmente eine mittlere Teilchengröße im Bereich von 0,5 bis 800 µm aufweisen.

6. Abdeckzusammensetzung nach Anspruch 5, **dadurch gekennzeichnet, dass** die Pigmente eine mittlere Teilchengröße (D50) von kleiner gleich 15 µm aufweisen.

7. Abdeckzusammensetzung nach einem der vorstehenden Ansprüche in Form eines Abdecksprays mit
1 bis 20 Gew.-% Polyisobutylen
2 bis 25 Gew.-% Pigment,
40 bis 80 Gew.-% Heptan,
10 bis 40 Gew.-% Treibmittel,
bis zu 2 Gew.-% Duftstoffen.

8. Abdeckzusammensetzung nach Anspruch 7, **dadurch gekennzeichnet, dass** sie Ethan, Propan, Butan, und/oder Dimethylether als Treibmittel enthält.

9. Abdeckzusammensetzung nach einem der vorstehenden Ansprüche, enthalten in einer Druckdose.

## Claims

1. Covering composition in the form of a spray for application to body surfaces, containing
- at least one water-resistant polymer in an amount of 1 to 20% by weight,
- at least one pigment in an amount of 2 to 25% by weight,
- at least one solvent for the water-resistant polymer in an amount of 40 to 80% by weight,
- up to 40% by weight of propellant,
- up to 40% by weight of additives,
wherein the data by weight are based on the total composition, **characterized in that** it contains polyisobutylene as the water-resistant polymer and **in that** the solvent is selected from acetone, isopropanol, pentane, hexane, heptane and/or methylal.

2. Covering composition according to any one of the preceding claims, **characterized in that** the polyisobutylene has a weight-average molecular weight of 5,000 to 5,000,000.

3. Covering composition according to claim 2, **characterized in that** the polyisobutylene has an average molecular weight of 35,000 to 100,000.

4. Covering composition according to any one of the preceding claims, **characterized in that** it contains pigments based on iron oxide, titanium dioxide and/or mica.

5. Covering composition according to claim 6, **characterized in that** the pigments have an average particle size of between 0.5 and 800 µm.

6. Covering composition according to claim 5, **characterized in that** the pigments have an average particle size (D50) less than or equal to 15 µm.

7. Covering composition according to any one of the preceding claims, in the form of a covering spray, comprising
1 to 20% by weight of polyisobutylene,
2 to 25% by weight of pigment,
40 to 80% by weight of heptane,
10 to 40% by weight of propellant,
up to 2% by weight of fragrances.

8. Covering composition according to claim 7, **characterized in that** it contains ethane, propane, butane and/or dimethyl ether as propellant.

9. Covering composition according to any one of the preceding claims, contained in an aerosol can.

## Revendications

1. Composition de masquage sous la forme d'un spray à appliquer sur des surfaces corporelles, contenant
- au moins un polymère résistant à l'eau en une quantité de 1 à 20 % en poids,
- au moins un pigment en une quantité de 2 à 25 % en poids,
- au moins un solvant pour le polymère résistant à l'eau en une quantité de 40 à 80 % en poids,
- jusqu'à 40 % en poids d'agent propulseur,
- jusqu'à 40 % en poids d'additifs,
les données en poids étant rapportées à la composition totale, **caractérisée en ce qu'**elle contient en tant que polymère résistant à l'eau du polyisobutylène et **en ce que** le solvant est choisi parmi l'acétone, l'isopropanol, le pentane, l'hexane, l'heptane et/ou le méthylal.

2. Composition de masquage selon l'une quelconque des revendications précédentes, **caractérisée en ce que** le polyisobutylène présente une masse moléculaire moyenne selon la moyenne en poids de 5 000 à 5 000 000.

3. Composition de masquage selon la revendication 2, **caractérisée en ce que** le polyisobutylène présente une masse moléculaire moyenne de 35 000 à 100 000.

4. Composition de masquage selon l'une quelconque des revendications précédentes, **caractérisée en ce qu'**elle contient des pigments à base d'oxyde de fer, de dioxyde de titane et/ou de mica.

5. Composition de masquage selon la revendication 6, **caractérisée en ce que** les pigments présentent une taille moyenne de particules comprise entre 0,5 et 800 µm.

6. Composition de masquage selon la revendication 5, **caractérisée en ce que** les pigments ont une taille moyenne de particules (D50) inférieure ou égale à 15 µm.

7. Composition de masquage selon l'une quelconque des revendications précédentes, sous la forme d'un spray de masquage avec
1 à 20 % en poids de polyisobutylène,
2 à 25 % en poids de pigment,
40 à 80 % en poids d'heptane,
10 à 40 % en poids d'agent propulseur,
jusqu'à 2 % en poids de parfum.

8. Composition de masquage selon la revendication 7, **caractérisée en ce qu'**elle contient de l'éthane, du propane, du butane et/ou de l'éther diméthylique en tant qu'agent propulseur.

9. Composition de masquage selon l'une quelconque des revendications précédentes, contenue dans une bombe aérosol.
